# EUROPEAN PATENT APPLICATION

(11) **EP 1 428 806 A1**
(43) Date of publication of application: **16.06.2004**
(21) Application number: 03078871.5
(22) Date of filing: 08.12.2003
(51) Int. Cl.: C04B 24/02, C04B 24/40, C04B 28/02

(54) **Concrete-based floors and wall coverings with an antimicrobial effect**

(30) Priority: 10.12.2002 MX 0212236
(71) Applicant: Cemex Trademarks Worldwide Ltd., 2555 Brugg (CH)
(72) Inventor: Ramirez, Tobias Homero, 64850 Monterrey Nuevo Leon (MX); De Leon, Guajardo Delia, 64850 Monterrey Nuevo Leon (MX)
(74) Representative: Davila Baz, Angel

(57) **Abstract**

A concrete-based floor or coating with anti-microbial effect, comprising a base material and an effective amount of at least one microbicide organic agent, mixed directly with the base material is described; the base material is a mixture containing cement, fine aggregates, solid aggregates, additives and water; where the microbicide agent mixed with the base material provides an efficient and long lasting anti-microbial effect.

## Description

### FIELD OF THE INVENTION

The present invention is related to construction materials having anti-microbial effect. More particularly, the present invention is related to floors and wall coverings made of cement, aggregates and additives, having effective and lasting anti-microbial effect.

### BACKGROUND OF THE INVENTION

Microbes control is a major concern on a wide variety of fields encompassing home living and several industries. For example, contamination or lack of hygiene on floors, walls and ceilings at homes is a microbial infection source under considerable moisture conditions within an enclosed room.

In the food industry, contamination with microorganisms in several stages such as: products manufacturing, processing and packing, is a serious problem from the point of view of food sanitation and preservation. In such case, the main microbial infection source is an environment contaminated by microorganisms that adhere and grow upon the room walls, ceilings and floors, where food manufacturing, processing and packing is carried out; this contaminating microorganisms may have the chance to spread out and contaminate the food.

Within the field of medical services, there exist strong demands for a biologically sanitized room as a measure against infections, due to an adverse environmental contamination of the surgery room, treatment room, examination room, patients room, waiting room, clinic, kitchen, etc. In this case, protecting walls, ceiling and floors against microbes attack and growth, represents a serious problem.

It is very important that medicines and drugs are produced within a microorganisms-free neat environment.

Within the field of the electronics industry, it is also acknowledged that manufacturing electronic components within a microbiologically neat environment is beneficial to improve electronic parts or components functioning, accuracy, reliability and production. Further, it is also important to prevent that bacteria and fungi adhere and grow on walls, ceilings and floors.

Conventional measures against contamination by microorganisms include: i) surface cleaning such as: room, area or ward floors, walls and/or ceilings, with cleaning agents such as detergent, bleach, etc., which shall be regularly carried out at relatively short periods; ii) applying a microbicide agent to the surface, which represents the disadvantage of having a sustained action and short period of the agent applied; and iii) applying a coating material incorporating a microbicide agent that is applied on the floor, wall and/or ceiling surface, coating material may be applied in the form of a layer, film or sheet.

Nowadays, a plurality of microbicide agents that can be used alone or combined to avoid microbe adhesion and spreading at homes and several industries surfaces has been developed. These microbicide agents can be mixed for example with cleaning products, compositions and disinfectants, or being applied as wall coverings on fabrics, paper, adhesive strips, plastic materials sheets, etc.

A particular example of the efforts made on the art is disclosed by the United States application No. 4,008,351 entitled "Sheet or film material having antibacterial and anti-fungal activity", issued on February 15, 1977 to Mayumi et al, which discloses a sheet or film material having antibacterial and anti-fungal activity utilized to cover walls, ceiling and floors. Said material is obtained by mixing a benzimidazole and phtalimide. Such sheet or film material disclosed by said application is a thermoplastic material based product, and even that can provide a protection against microbial growth, it has the disadvantages of being an additional component which is submitted to wearing due to use and can be coated or replaced at the short run with the additional expenses entailed. This application does not suggest that microbicide agents can be an integral part of cement and aggregates based composition. Equally, making a long lasting product, such as floor or coating having a lasting anti-microbial effect, is not suggested.

United States application No. 6,162,845 entitled "Reinforced Concrete having Improved Anti-microbial Fibers", issued on December 19, 2000, to W. Wayne Freed, discloses a material product similar to concrete reinforced with fibers having an effective amount of a microbicide agent incorporated therein, to prolong concrete duration, where the microbicide agent is provided incorporated in or coating a plurality of fibers, which, in turn, are spread within a concrete matrix. Fibers inclusion with a microbicide agent is aimed to reinforce concrete and reduce concrete sensitivity to a biological attack. However, this document is referred to the fiber reinforced concrete specific application, whose fiber includes a microbicide agent (Microban ® B) that protects concrete itself from deterioration due to microorganisms growth, this concrete is used for the construction of entire buildings and specially those in which an additional protection due to the use of this kind of agents is required. However, reference is not made to concrete-based floors and wall coverings in different forms and designs, that do not include a fiber as a reinforcement element and as a carrier for incorporating a microbicide agent, for the anti-microbial protection when being utilized as a floor and coating for walls and ceilings without the need of the entire building construction with such products. Also, there is no reference made of a finished product for specific use, having proved long lasting anti-microbial action using microbicide agents without including a fiber as a carrier.

United States application No. 6,350,350 entitled "Construction Material", issued on February 26, 2002 to Eisaku Tozaka, discloses a construction material containing microcapsules of a specific bactericide agent, mixed with a basic construction material such as cement, aggregates and water mixture. Said basic material with the bactericide agent microcapsules is used for floors, walls and ceilings construction. The liquid bactericide agent contained in the microcapsules is gradually perspired or infiltrated into the construction material each time microcapsules break due to a change over time. The construction material with an antibacterial action of the present invention presents the disadvantage of requiring further devices and processes for first forming the microcapsules containing the specific microbicide agent, and after having obtained this microcapsules, are incorporated with the mixture to finally form the construction material. Another disadvantage of this construction material is the cost factor entailed when being used to build walls and construction in general, to provide the protection mentioned above. However, there is no suggestion on said application, that the microbicide agent be directly integrated as another component of the construction material, and including an immediate and long lasting anti-microbial action, this is, without using a carrier such as microcapsules.

There are other publications and applications that incorporate several additives being mixed with concrete or a construction material, or being disposed as a coating on said concrete or construction material. For example, Russian application No. 2149147, entitled "Agglomerating Agent" to Sycheva et al., published on May 20, 2000 discloses the preparation of solutions and concretes under low temperatures showing an antibacterial activity by the incorporation of KmnO₄ to a ratio of 0.1 to 0.3% by weight. The use of KmnO₄ as a microbicide compound shows the disadvantage of providing a particular coloration to the concrete, preventing the production of several products with different colors. Besides, due to said coloration provided by KmnO₄, its application on floors and ceilings and walls wall coverings is very limited.

Japanese application No. 11236734 entitled "Antibacterial Construction Material" to Miyasaki Norihiko published on August 31, 1999, discloses an ultra-violet curing type antibacterial coating composition for interiors finishing, such as a floor, wall or ceiling; where the antibacterial coating composition is a painting-type composition comprising a base resin and amino acid salt of an antibacterial metal. This kind of wall coverings show the disadvantage of metal-based products used for providing the antibacterial effect, such metal-based products present problems, as its handling and application to form small layers or protective films is concerned. For the particular case that is used as a coating for a floor, said coating is worn or removed due to simple use, being necessary to prepare again the floor to apply the antibacterial coating composition once again.

Chinese application No. 1223795 entitled "Bactericide Additive for Construction, its Preparation Method and Use" to Tian Shulin, published on July 28, 1999, discloses a bactericide additive for construction materials which is prepared from a carrier and antibacterial metal salt. This document shows the disadvantage that additional stages and materials for manufacturing and adequate bactericide additive that can be used for the construction industry are required. However, it is not mentioned that said active component could be directly used for preparing concrete-based elements (for example, floors, walls, and ceilings coating) keeping its microbicide properties and sustaining a long lasting effect.

Japanese application No. 4331752 entitled "Antibacterial and Anti-fungal Basic Material for Construction" to Nawata Nozomi et al., published on November 19, 1992 is referred to the addition of 0.01 to 30 parts by weight of an antibacterial and anti-fungal compound, such as 2,2,3-thioyodineallyl alcohol added to the base material (cement or plaster). The antibacterial and anti-fungal compound can be mixed with a base material, where the base material is in powder, not molded or not cured yet; this antibacterial and anti-fungal compound is applied to the base material surface through the recoating or spraying method. This Japanese application describes a product with an antibacterial and anti-fungal action which is used to coat with small layers the elements desired to be protected; however, it does not suggest the use of a microbicide agent in an integral form of the concrete-based element used on floors, walls, and ceilings wall coverings, etc., which has a long lasting anti-microbial effect and which does not require any further protecting layers for protecting the surface.

Japanese application No. 8109108 entitled "Construction material having an antibacterial activity consisting of a combined material obtained by using mordenite and its production" to Ogura Ywsuke et al., published on April 30, 1996, discloses a material that comprises a carrier made from mordenite (main component) that is immersed in a diluted solution of silver nitrate, where an alkaline ion is displaced with a silver ion. The carrier is in combination with a construction material, such as cement, wood, chips, paper or fibers to obtain a combined material. This combined material is formed and processed desirably as a construction material. This material is useful indoors as well as outdoors. This Japanese application is related to a material for general construction, which includes a further step of soaking one of its components with the antibacterial agent solution to displace one alkaline ion with a silver ion, however, it is not mentioned that the agent can be directly added without the use of a carrier to dose the microbicide agent, avoiding the use of heavy metal based compounds and the need of additional steps, providing an efficient and long lasting anti-microbial effect.

According to the above mentioned, there is no document registered that describes or suggests a concrete-based floor or coating not requiring additional materials or carriers and procedures to directly incorporate the microbicide agent as a component of the concrete-based floors and wall coverings composition. Consequently, there is a need for useful concrete-based floors and wall coverings for applications where a more hygienic environment is required, that comprises at least an organic microbicide agent being readily mixed directly, and with fast and long lasting release of the active agent on its surface, to inhibit microbial growth.

Therefore, it is an objective of the present invention to provide common manufactured concrete-based floors and wall coverings, and at the same time exhibit an efficient and long lasting anti-microbial effect.

Another objective of the present invention is to provide at least one microbicide agent as a cement component, aggregates and additives mixture, for manufacturing finished floors and wall coverings ready to be installed.

Still another objective of the present invention is to provide concrete-based floors and wall coverings with efficient inhibition of microorganisms growth and contact, to be used in areas such as homes, several industries, food processing plants, pharmaceutical labs, hospitals, etc., which require extraordinary cleanliness.

Still a further objective of the present invention is to provide a low cost finished product of the type of a concrete-based floor and/or coating with anti-microbial effect that will not require the use of carriers to be incorporated into a base material without special mechanisms for its release.

### DETAILED DESCRIPTION OF THE INVENTION

Generally, the present invention is related to a floor and/or coating with an anti-microbial effect, being concrete-based manufactured and which includes at least a microbicide agent that provides an efficient and long lasting effect.

In one embodiment of the invention, the concrete-based floor and/or coating with anti-microbial effect comprises a base material and an effective amount at least of a microbicide organic agent mixed directly with the base material. The base material is a mixture comprising cement, fine aggregates, solid aggregates, additives and water.

In another aspect of the invention, the floor and/or coating base material comprises from about 5 to approximately 50% by weight of cement, up to approximately 50% by weight of fine aggregates, from about 20 to approximately 30% by weight of solid aggregates, from about 0.01 to approximately 5% by weight of additives, and where a 0.01% to 5% by weight of a base material of at least one microbicide agent or a mixture of microbicide agents is added.

### DETAILED DESCRIPTION OF THE PREFERRED EMBODIMENTS

As mentioned before, the present invention is related to a concrete-based floor and/or coating which includes an effective amount at least of a microbicide agent or a mixture of microbicide agents to inhibit biological attack and spreading out of organisms.

The present invention is a concrete-based construction material for finishes such as a floor and/or coating having an immediate anti-microbial effect. The concrete-based construction material for finishes comprises a base material and an effective amount at least of a microbicide organic agent or a mixture of various microbicide organic agents being directly mixed with the base material. The base material is a mixture comprising cement, fine aggregates, solid aggregates, additives and water. In a preferred embodiment of the present invention, the base material comprises from about 5% to approximately 50% by weight of cement, up to approximately 50% by weight of fine aggregates, from about 20 to approximately 30% by weight of solid aggregates, from about 0.01 to approximately 5% by weight of additives.

Useful microbicide agents in the present invention are organic, non-toxic, and environmentally save during their use. As it is known, most of microbicide agents control the organisms growth by penetrating the organism thin cellular walls, thereby interrupting the organism metabolic function, and finally killing said organism. In this way, it shall be understood in the present invention that the term "microbicide agent" refers to including any organic compound that effectively inhibits a microbial attack and its spreading out on a concrete-based finished floor or coating. The term "at least one microbicide agent" as used in the present invention, should be understood as been feasible to use a microbicide agent or a mixture of different microbicide agents.

Proper microbicide agents to be used by the present invention are derivatives from phenol, especially 2,4,4'-trichlorine-2'-hydroxyphenol (for example, Triclosan, irgasan, microban), organotins, especially butyl-tin maleate (for example, Ultra Fresh), sodium o-phenylphenate tetrahydrate (Dowcide-A), and alkyldimethyl benzylammonium chloride.

Dose ratio for adding microbicide agents to the base material used by the present invention varies significantly depending on the use and environmental conditions of the place on which the concrete-based floor or coating will be applied. Therefore, an effective amount of a microbicide agent to prevent bacteria and microorganisms adhesion, growth and spreading out at homes and several industries surfaces is required. In most of the cases, the effective ratio of the microbicide agent or the mixture of microbicide agents will range from 0.01% to 5% by weight of the base material.

A preferred method to add at least a microbicide agent is simply mixing the microbicide agent along with the base material components, this is, cement, fine aggregates, solid aggregates, additives and water, through conventional means well known in the state of the art. There for, a concrete mixture with anti-microbial effect is obtained, which is subsequently submitted to a manufacturing procedure to form a floor and/or coating.

An illustrative manufacturing method to form a floor and coating comprises the following steps:
1) Dose ratio.- Base material ingredients or components at an adequate ratio, being according to the required resistance design by the application that shall be given to the element, are dosed along with an effective amount of at least one microbicide organic agent, depending on the anti-microbial effect desired. Main base material compounds are cement, fine aggregates, solid aggregates, (marble and granite pieces, etc.), dyes and water.
2) Mixing or kneading.- Once the base material and the microbicide agent being dosed, these are mixed or kneaded, through any appropriate method or device known in the art, to obtain a homogeneous paste with anti-microbial effect and properly integrate the base material ingredients and the microbicide agent.
3) Moulds refilling.- An adequate amount of previously homogenized paste with anti-microbial effect is dosed and refilled to the moulds, which can have several forms and sizes according to the floors and wall coverings design (rectangular, curved, etc.).
4) Pressing.- The paste previously poured into the moulds is submitted under pressure application, through a press, for the paste to eliminate water excess and to obtain a product with the adequate density and porosity to be handled by the machinery for subsequent stages.
5) Curing.- Once the product is hardened through the pressing stage, floors and wall covering frames are disassembled, and taken to the curing room; the latter can be made under normal temperature, which will last 48 hours or by using vapor, through which time is reduced to 24 hours.
6) Calibration.- Once floor tiles have been set and acquired a resistance through the curing stage, their dimensions are calibrated by using surfacing and shaping machines perfectly aligned.
7) Polishing and brightening.- After calibration, floors and wall coverings pass through polishing and brightening line, which comprises multiple grindstones which boast, abrade, polish and brighten the above.
8) Packing.- final product is packed in multiple presentations.

### EXAMPLE

In order to show the practice of the invention and evaluate the efficacy thereof, a base material is prepared by mixing 25% by weight of cement, 50% by weight of fine aggregates, 24.5% by weight of solid aggregates (marble pieces), and water in a sufficient amount to form a mixture. During base material mixture forming, a microbicide agent of 0.05% by weight (Triclosan) is added and homogeneously mixed with the entire mixture of base material, forming thereby a base material with anti-microbial effect. Then, the mixture with anti-microbial effect is poured in a mould with adequate shape and dimensions in order to form a concrete floor tile.

After the floor tile with anti-microbial effect has been cured, a sample of approximately 25 mm of diameter (treated element) is taken to prove the anti-microbial inhibition along with the floor tile made with a non-treated conventional material (element of control). Test results are shown in table 1 below.

**TABLE 1**

| **Microorganism** | **Control element** | **Treated element** | **Inhibition zone** |
|---|---|---|---|
| *Escherichia coli* | Growth | No growth | 5 mm |
| *Staphilococus aureus* | Growth | No growth | 17 mm |
| *Enterobacter aerogenes* | Growth | No growth | 5 mm |

As it can be seen from table 1, the inhibition zone around test tubes treated with the microbicide agent (Triclosan in this case) shows the effect the elements have when treated to inhibit microorganisms growth and reproduction, in other tests, the growth inhibitor effect that this kind of treatment has on fungi, has also been shown. This shows the protecting capacity these concrete-based floors and wall coverings have to contribute for the protection of microorganisms spreading out on places where they have been used.

Even though certain embodiment of the invention has been illustrated and described, it should be noted that several changes and modifications could be made there on. There for, the present invention shall not be considered as limited excepting by what the prior art demands and by the spirit of claims attached herein.

## Claims

1. Concrete-based floors and wall coverings with anti-microbial effect, made on a concrete basis, wherein they comprise a base material and an effective amount of at least one microbicide organic agent that provides an efficient and long lasting effect.

2. Concrete-based floors and wall coverings with anti-microbial effect, made on a concrete basis, according to claim 1, wherein said at least one microbicide agent forms an integral part of the mixture used for floors and wall coverings manufacturing.

3. Concrete-based floors and wall coverings with anti-microbial effect, made on a concrete basis, according to claim 2, wherein said base material comprises from about 5 to approximately 50% by weight of cement, up to approximately 50% by weight of fine aggregates, from about 20 to approximately 30% by weight of solid aggregates, from about 0.01 to approximately 5% by weight of additives, and where the microbicide agent is present in an amount of about 0.01% to 5% by weight of the base material.

4. Concrete-based floors and wall coverings with anti-microbial effect, made on a concrete basis, according to claim 2, wherein said microbicide organic agent is selected from a group consisting of derivatives of phenol, organotins, sodium o-phenylphenate tetrahydrate or alkyldimethyl benzylammonium chloride or a mixture thereof.

5. Concrete-based floors and wall coverings with anti-microbial effect, made on a concrete basis, according to claim 4, wherein said phenol derivatives are preferably derived from 2,4,4'-trichlorine-2'-hydroxyphenol, and said organotin is preferably butyl-tin maleate.

6. A method to manufacture floors and wall coverings with anti-microbial effect of the type that comprises: a) dose ratio a base material comprised of cement, fines aggregates, solid aggregates, additives and water, on the adequate ratios according to the resistance design required for the application that will be given to the floor or coating; b) mixing base material components to form an homogenous paste; c) pouring homogeneous paste in adequate shape and size moulds according to the floors and wall coverings design; d) pressing the homogeneous paste contained in the moulds to eliminate the water excess and obtain a dewatered paste with the adequate density and porosity; e) the frame disassembly dewatered homogenous paste from the mould; f) curing the dewatered homogenous paste under at an environment temperature during 48 hours or through the use of water vapor during 24 hours to obtain a concrete-based, cured floor or coating; g) calibrating the cured floor or coating to boast and shape its dimensions; and h) polishing and brightening concrete-based floor or coating; wherein it comprises a further step of adding an effective amount of at least one microbicide agent before the step b.

7. The method of manufacturing floors and wall coverings with anti-microbial effect according to claim 6, wherein said at least microbicide agent forms an integral part of the paste used for said floors and wall coverings manufacturing.

8. The method of manufacturing floors and wall coverings with anti-microbial effect according to claim 7, wherein said effective amount of the microbicide agent will depend on the use and environmental conditions of the place on which the concrete-based floor or coating will be applied; to prevent a bacteria or microorganisms adhesion growth and spreading out upon said floors and wall coverings surfaces.

9. The method of manufacturing floors and wall coverings with anti-microbial effect according to claim 8, wherein said effective amount of the microbicide agent will range from 0.01% to 5% by weight of the base material.
